Europäisches Patentamt

European Patent Office

Office européen des brevets

(11) Publication number: **0 225 940**
**A1**

## EUROPEAN PATENT APPLICATION

(21) Application number: 85116407.9

(22) Date of filing: 20.12.85

(51) Int. Cl.⁴: **D21C 9/00** , D21B 1/06 , A61L 15/00

(43) Date of publication of application:
24.06.87 Bulletin 87/26

(84) Designated Contracting States:
AT BE CH DE FR GB IT LI LU NL SE

(71) Applicant: **Scamvougeras, Maria**
**127, Patission Street**
**Athene 11251(GR)**

(72) Inventor: **Scamvougeras, Maria**
**127, Patission Street**
**Athene 11251(GR)**

(74) Representative: **Strulf, Bernward, Dipl.-Chem.**
**Dr. et al**
**Patentanwaltsbüro Tiedtke, Bühling, Kinne**
**Grupe, Pellmann, Grams, Strulf Bavariaring 4**
**D-8000 München 2(DE)**

(54) Process for the production of disposable hygienic goods and fluff pulp for using in this process.

(57) A process for the production of disposable hygienic goods made of fluff pulp is described wherein untreated fluff pulp is defibred in a hammer mill and sucked through a screen. The defibration is carried out in presence of a lubricant by which the power consumption of the hammer mill can be decreased. Fluff pulp containing a lubricant can be used in the above process.

EP 0 225 940 A1

## PROCESS FOR THE PRODUCTION OF DISPOSABLE HYGIENIC GOODS AND FLUFF PULP FOR USING IN THIS PROCESS

The invention relates to a process for the production of disposable hygienic goods made of fluff pulp and to fluff pulp used in this process.

Fluff pulp is the main raw material for the production of disposable hygienic goods. Depending on the kind of treatment fluff pulp can be divided in two groups, namely treated and untreated fluff pulp. Treated fluff pulp is obtained when a surfactant is added during the pulping step, to break the binding of the molecule structure of the fibres. Normally a cationic surfactant like a quaternary ammonium salt of long chain dialkylamines is used. Untreated fluff pulp is obtained without treating with such a surfactant. The two different kinds of fluff pulp can be used for defibration in a hammer mill for the production of disposable hygienic goods.

When treated fluff pulp is defibred in a conventional hammer mill the fluff pulp is cut in relatively short fibres which do not cause any problem in the hammer mill, but such short fibres do not absorb sufficiently moisture, so that, when using the fibres of treated fluff pulp for the production of hygienic goods the moisture or liquid will be concentrated in a small portion thereof. This causes skin irritation and discomfort. Therefore, by using treated fluff pulp a product with law quality is obtained.

When untreated fluff pulp is defibred in a conventional hammer mill relatively long fibres result. Such long fibres absorb liquid or moisture easier and diffuse it faster in larger surface portions of the hygienic product, causing a good skin compatibility and a high comfort. Therefore, the use of untreated fluff pulp results in the production of hygienic products with better quality.

During the defibration in a hammer mill untreated fluff pulp however causes the following problems:

After the defibration of the fluff pulp in the hammer mill the defibred fluff pulp is sucked out of the hammer mill through a screen with openings having different profiles depending on the desired grade of defibration. The size of the openings determines the period of time in which the fluff pulp is in the hammer mill and thus the grade of defibration, so that the size of the openings for optimal defibration is limited.

With long fibres, however, there is the risk that the fibres accumulate at the openings, i.e. these may be closed, so that the resistance of motion of the hammer mill increases, which means that for maintaining the productivity more driving energy has to be supplied. Consequently a higher temperature is developed within the hammer mill, which may result in accelerated wear and the breaking of the screen (with the risk of fire). The problem may be solved by widening the openings, but this would lead to a deteriorated product because the period of time in which the fibres are in the hammer mill is shortened.

Object of the present invention is to provide a process for the production of disposable hygienic goods in a hammer mill by which the defibred fluff pulp can easier pass the openings of the screen, so that untreated fluff pulp can be used as raw material whereby at least the same productivity of the hammer mill is achieved than by using treated fluff pulp.

A further object of the present invention is to improve a fluff pulp which can be used in the above mentioned process.

The present invention comprises a process for the production of disposable hygienic goods made of fluff pulp by defibrating untreated fluff pulp in a hammer mill and sucking the fibred fluff pulp through a screen, wherein the defibration is carried out in the presence of a lubricant.

Further the invention comprises a fluff pulp for the production of disposable hygienic goods, wherein the fluff pulp contains a lubricant and is used in the above-mentioned process.

According to the invention the following advantages can be achieved:

1) The productivity of the hammer mill when using untreated fluff pulp is least the same, when using treated fluff pulp, without causing stuffing problems within the hammer mill.

2) The power consumption of the hammer mill at a given feed of fluff pulp is decreased to about 30 - 60% when using a lubricant in comparison with a treatment without lubricant.

3) Due to the reduction of the friction less wear of the hammers, ball bearings, screen, motors etc. occurs.

The present invention is based on the finding that the defibration can be carried out in the presence of a lubricant. It is assumed that the lubricant results in lubricating the inner side during the passage of the defibred fluff pulp, so that the fluff pulp does not stick on the inner sides of the screen, so that the defibred fluff pulp can be sucked out of the hammer mill easier.

According to an embodiment of the present invention the lubricant is a lubricating oil, which is liquid at the defibration temperatures. The defibration is normally carried out at 30 to 110°C especially at 60 to 100°C. Suitable lubricants are chemically inert and compatible for the human skin. The lubricant can be selected from the group mineral oils, animal oils, vegetable oils, lubricating ether and silicone oils. Preferred lubricants are hydrocarbon oils, especially paraffin oils, polyglycols, especially ethylene glycols, propylen glycols, esters and ethers thereof, vaseline and glycerides of fatty acids. Examples for suitable lubricants are paraffin baby oil and non-drying glyderides of fatty acids like olive oil. Further mixtures of the above-mentioned lubricants can be used.

The lubricant is used in an amount of at least 0.1 g per 1 kg fluff pulp, preferably in an amount of 1 g to 25 g, especially 1 g to 10 g per 1 kg fluff pulp. When the lubricant is paraffin baby oil or a non-drying vegetable oil, like olive oil it is used preferably in an amount of 1 g to 5 g, especially 2 g to 4 g per 1 kg fluff pulp.

The lubricant is added to the fluff pulp before or during the defibration. The lubricant can be added to the fluff pulp e.g. by spraying or by another suitable procedure.

The hammer mill for defibration of the fluff pulp is a conventional one. Such a conventional hammer mill comprises a lot of rotating hammers and a screen with openings through which the defibred fluff pulp is sucked out. The screen has normally openings of 5 to 20 mm preferably 8 to 12 mm and especially 9 to 10 mm wherein the openings are preferably round holes. The fluff pulp is introduced into the hammer mill in a conventional manner.

Normally untreated fluff pulp having a weight per unit area of 450 to 900 g/m$^2$ is used. During the defibration, if necessary, water is added in order to adjust the humidity of the fluff pulp to at least 4%, based on the weight per unit area. In this case water can be sprayed into the hammer mill.

The lubricant can be removed after the defibration process. In a preferred embodiment the lubricant remains unchanged in the defibred fluff pulp if the lubricant is compatible for human skin. In this case the lubricant can be added in a sufficient amount to the fluff pulp, so that a care effect in the finished disposable hygienic product can be achieved. Further it is possible to use an odourant together with the lubtricant in order to perfume the disposable hygienic products.

According to a further embodiment the invention comprises fluff pulp containing a lubricant which is used in a process as described above. The lubricant is especially a lubricating oil, which is liquid at 30°C or above and in particular at 60°C or above. Suitable lubricants are chemically inert and compatible for the human skin. The lubricant can be selected from the group mineral oils, vegetable oils, animal oils, lubricating ether and silicone oils. Preferred lubricants are hydrocarbon oils, especially paraffin oils, polyglycols, especially ethylene glycols, propylen glycols, esters and ethers thereof, vaseline and glycerides of fatty acids. Examples for suitable lubricants are paraffin baby oil, and non-drying glycerides of fatty acids like olive oil. Further mixtures of the above-mentioned lubricants can be used.

The lubricant is used in an amount of at least 0.1 g per 1 kg fluff pulp, preferably in an amount of 1 g to 25 g, especially 1 g to 10 g per 1 kg fluff pulp. When the lubricant is paraffin baby oil or an non-drying vegetable oil, like olive oil it is used preferably in an amount of 1 g to 5 g, especially 2 g to 4 g per 1 kg fluff pulp.

The fluff pulp can be untreated or treated fluff pulp, wherein untreated fluff pulp is preferred. The weight per unit area of the fluff pulp is about 450 to 900 g/cm$^2$. The fluff pulp can contain water in an amount of at least 4% based on the weight per unit area.

The lubricant can be added in any step of the pulping procedure, especially before, during or after the sheet forming and drying step or the roll and bale finishing step. The lubricant can also be added before the defibration step. The addition of the lubricant can be carried out by spraying, impregnating, dipping or by another suitable prodecure. The thus obtained fluff pulp in the form of sheets or rolls can then be introduced in the hammer mill as described above.

The invention is further illustrated by way of examples.

Example 1

For the defibration of fluff pulp a conventional hammer mill with 108 hammers and a motor having 88,25 kW is used, which can be operated at 3000 revolutions per minute. The screen openings of the hammer mill have a diameter of about 9 mm. The capacity of the whole apparatus is designed for the production of about 300 pieces of the finished product per minute.

Untreated fluff pulp is used in form of rolls having a width of 33 cm. The amount of fluff pulp fed into the hammer mill is varied according to the table below. Water is sprayed, if necessary, into the hammer mill, in order to ensure a sufficient humidity of the fluff pulp (4 to 6%, based on the weight per unit area of the fluff pulp). The fluff pulp is defibred in absence of a lubricant, or in presence of a surfactant or in presence of different lubricating oils at various amounts, as given in the table below. The amperage of the hammer mill is measured depending on the different conditions as mentioned in the following table:

Table

| Feed of fluff into the hammer mill (kg/min.) | 5.2 | 6 | 6.9 | 7.7 | 8.6 | 9.5 |
|---|---|---|---|---|---|---|
| | | | Amperage | | | |
| Without lubricant | 55 | 60 | 71 | 79 | 88 | 98 |
| Olive oil (2.3 g/kg fluff) | 52 | 56 | 68 | 75 | 85 | 90 |
| Olive oil (4.5 g/kg fluff) | 40 | 45 | 52 | 58 | 65 | 70 |
| Silicon oil[1] (2 g/kg fluff) | 40 | - | 40 | - | 55 | 70 |
| Paraffin oil (Baby oil) (2 g/kg fluff) | 40 | 40 | 40 | 50 | 55 | 70 |
| Polyethylene glycol[2] (2.5 g/kg fluff) | 40 | - | 40 | - | 54 | 68 |
| Paraffin oil (Baby oil) (4 g/kg fluff) | 40 | 40 | 40 | 45 | 50 | 60 |
| Surfactant (Pril®) (2 to 10 g/kg fluff) (comparison) | 53 | 58 | 70 | 77 | 86 | 96 |

1) sold by Dow Chemical under type corning 200 having fluid viscosity 350
2) sold by Shell

From the table it can be gathered that by using lubricating oils the amperage of the hammer mill can be decreased to about 40 to 60% of the amperage of the hammer mill when no lubricant is used. That means the productivity of the hammer mill at a given amperage can be increased to about 40 to 60% of that when using a lubricant. The use of a surfactant has practically no influence on the amperage of the hammer mill, so that surfactants are unsuitable.

## Claims

1. Process for the production of disposable hygienic goods made of fluff pulp by defibring untreated fluff pulp in a hammer mill and sucking the defibred fluff pulp through a screen, characterized in that the defibration is carried out in presence of a lubricant.

2. Process according to claim 1, wherein the lubricant is a lubricating oil, which is liquid at the defibration temperatures.

3. Process according to claim 1 or 2, wherein the lubricant is chemical inert and compatible for the human skin.

4. Process according to anyone of the preceding claims, wherein the lubricant is used in an amount of at least 0.1 g per 1 kg fluff pulp.

5. Process according to anyone of the preceding claims, wherein the lubricant is used in an amount of 1 g to 25 g, especially 1 g to 10 g per 1 kg fluff pulp.

6. Process according to anyone of the preceding claims, wherein the lubricant is selected from the group mineral oils, animal oils, vegetable oils, lubricating ether and silicone oils.

7. Process according to anyone of the preceding claims, wherein the lubricant is selected from the group hydrocarbon oils, especially paraffin oils, polyglycols, especially polyethylene glycols, polypropylen glycols, esters and ethers thereof, vaseline, and glycerides of fatty acids.

8. Process according to anyone of the preceding claims wherein the lubricant is a paraffin baby oil or a non-drying vegetable oil, especially olive oil which is used in an amount of 1 g to 5 g, especially 2 g to 4 g per 1 kg fluff pulp.

9. Process according to anyone of the preceding claims, wherein the defibration is carried out at 30 to 110°C, especially at 60 to 100°C.

10. Process according to anyone of the preceding claims, wherein the untreated fluff pulp has a weight per unit area of 450 to 900 g/m².

11. Process according to anyone of the preceding claims, wherein during the defibration if necessary water is added in order to adjust the humidity of the fluff pulp to at least 4% base on the weight per unit area.

12. Process according to anyone of the preceding claims, wherein the screen has openings of 5 to 20 mm preferably 8 to 12 mm, and in particular 9 to 10 mm.

13. Process according to claim 12, wherein the openings are round holes.

14. Process according to anyone of the preceding claims, wherein the lubricant is added to the fluff pulp before or during the defibration.

15. Process according to anyone of the preceding claims, wherein the lubricant is removed after the defibration.

16. Fluff pulp for the production of disposable hygienic goods, characterized in that the fluff pulp contains a lubricant and is used in a process according to anyone of the preceding claims.

17. Fluff pulp according to claim 16, wherein the lubricant is a lubricating oil, which is liquid at 30°C or above and especially at 60°C or above.

18. Fluff pulp according to claim 16 or 17 , wherein the lubricant is a lubricating oil, which is chemical inert and compatible to the human skin.

19. Fluff pulp according to claims 16 to 18, wherein the amount of the lubricant is at least 0.1 g per 1 kg fluff pulp.

20. Fluff pulp according to claims 16 to 19, wherein the amount of lubricant is 1 to 25 g, especially 1 to 10 g per 1 kg fluff pulp.

21. Fluff pulp according to claims 16 to 20, wherein the fluff pulp is untreated or treated fluff pulp.

22. Fluff pulp according to claims 16 to 21, wherein the weight per unit area of the fluff pulp is 450 to 900 g/m².

23. Fluff pulp according to claims 16 to 22, wherein the fluff pulp contains water in an amount of at last 4% based on the weight per unit area.

24. Fluff pulp according to claims 16 to 23, wherein the lubricant is selected from the group mineral oils, vegetable oils, animal oils, lubricating ethers and silicone oils.

25. Fluff pulp according to claims 16 to 24, wherein the lubricant is selected from the group hydrocarbon oils, especially paraffin oils, polyglycols, especially polyethylene glycols, polypropylen glycols, esters and ethers thereof, vaseline, and glycerides of fatty acids.

26. Fluff pulp according to claims 16 to 25, wherein the lubricant is paraffin baby oil in an amount of 1 g to 5 g, especially 2 g to 4 g per 1 kg fluff pulp.

27. Fluff pulp according to claims 16 to 26, wherein the lubricant is an non-drying vegetable oil, especially olive oil in an amount of 1 g to 5 g, especially 2 g to 4 g per 1 kg fluff pulp.

28. Fluff pulp according to claim 16 to 27, wherein the lubricant is added before, during or after the sheet forming and drying steps or the roll and bale finishing step or before the defibration step.

# EUROPEAN SEARCH REPORT

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| X | US-A-3 591 450 (J.A. MURPHY et al.)<br><br>* Figures 1-4; columns 1,2; column 3, lines 1-29; column 6, line 39 - column 7, line 65 *<br><br>--- | 1-7,9, 11,14- 21,23- 25,28 | D 21 C 9/00<br>D 21 B 1/06<br>A 61 L 15/00 |
| X | US-A-3 930 933 (D.K. GEORGE et al.)<br><br>* Column 1, lines 32-38 *<br><br>--- | 1-3,6, 7,14, 16-19, 24,25, 28 | |
| A | US-A-4 022 861 (Y.G. LEVESQUE)<br><br>--- | | |
| A | US-A-4 241 881 (E.P. LAUMER)<br><br>--- | | TECHNICAL FIELDS SEARCHED (Int. Cl.4)<br><br>A 61 L<br>D 21 B<br>D 21 C<br>D 21 H |
| A | US-A-3 702 801 (M.L. DE MATTE)<br><br>--- | | |
| A | GB-A-2 033 751 (JOHNSON & JOHNSON)<br><br>--- | | |
| A | GB-A-1 131 762 (COLGATE & PALMOLIVE)<br><br>--- -/- | | |

The present search report has been drawn up for all claims

| Place of search | Date of completion of the search | Examiner |
|---|---|---|
| THE HAGUE | 14-08-1986 | NESTBY K. |

| | DOCUMENTS CONSIDERED TO BE RELEVANT | | | Page 2 |
|---|---|---|---|---|
| Category | Citation of document with indication, where appropriate, of relevant passages | | Relevant to claim | CLASSIFICATION OF THE APPLICATION (Int Cl 4) |
| A | GB-A- 720 363 (TAMPAX) | | | |
| | --- | | | |
| A | FR-A-1 548 038 (THE PROCTER & GAMBLE CO.) | | | |
| | ----- | | | |
| | | | | TECHNICAL FIELDS SEARCHED (Int. Cl.4) |

The present search report has been drawn up for all claims

| Place of search | Date of completion of the search | Examiner |
|---|---|---|
| THE HAGUE | 14-08-1986 | NESTBY K. |

EPO Form 1503 03 82